# EUROPEAN PATENT SPECIFICATION

(11) **EP 2 512 569 B1**
(45) Date of publication and mention of the grant of the patent: **22.01.2014**
(21) Application number: 10792913.5
(22) Date of filing: 15.12.2010
(51) Int. Cl.: A61M 16/04, A61M 25/09

(54) **PERFORATOR AND ASSEMBLY FOR PERFORATING AND PROVIDING AN OPENING**
PERFORATOR UND EINHEIT ZUM PERFORIEREN UND BEREITSTELLEN EINER ÖFFNUNG
PERFORATEUR ET ASSEMBLAGE POUR PERFORER ET FOURNIR UNE OUVERTURE

(30) Priority: 15.12.2009 EP 09179308; 15.12.2009 US 286561 P
(43) Date of publication of application: 24.10.2012
(73) Proprietor: Danmarks Tekniske Universitet, 2800 Lyngby (DK)
(72) Inventor: KLIT, Peder, DK-3480 Fredensborg (DK); KLIT, Anders, DK-1810 Frederiksberg C (DK)
(74) Representative: Zacco Denmark A/S
(86) International application number: PCT/EP2010/069740
(87) International publication number: WO 2011/073249

(56) References cited:
- GB-A- 2 428 201
- US-A- 5 578 053
- US-A- 5 681 323
- US-A1- 2009 163 942

## Description

### Technical field

The invention relates to a hole stabilizer used together with a perforator for providing and maintaining an opening through a patients skin surface thereby providing short or long-term external access to an inner air-filled cavity of a patient.

Establishing a tracheotomy is necessary if the airways are blocked or obstructed e.g. due to trauma, infection, different types of allergy resulting in oedema and swelling in the throat or e.g. foreign objects stuck in the throat. All conditions which can lead to asphyxia. If such an emergency appears it might be necessary to perforate the wall of the trachea in order to allow the patient to breathe. Normally this procedure is performed by skilled persons such as emergency physicians and surgeons and the percutaneous technique is used globally.

### Background of the invention

US 4.556.059 relates to a device for automatic mechanical performance of an emergency tracheotomy. An incision into the trachea is made upon release of a power-operated cannula with a cutting blade by a triggering mechanism. The device consists of a hollow cannula with a cutting blade on the frontal edge retained in a hollow, elongated housing. The cutting blade penetrates the tissues and trachea allowing entry of the cannula into the trachea. The cannula and housing provide an immediate temporary channel for air into the trachea and permit a breathing tube to be inserted through them into the trachea. The cannula has a curved portion in its leading edge to direct the insertion of the breathing tube in a downward direction into the trachea to prevent damage to the posterior wall of the trachea. The blade and housing may then be withdrawn over the breathing tube and discarded. The breathing tube is then secured to maintain the air passageway. According to description of this device the act of cutting and the insertion of a breathing tube are two separate actions.

US 6.971.382 relates to a device for a trachea tube having an interior positioned retaining member and a flange forming an exteriorly position retaining member. The trachea tube has means that permits the tube to be held firmly in the proper position upon a person on whom the device is installed. The tube maintains the stoma open.

This tracheotomy tube is provided an inflatable element that permits the tube to be held firmly in the proper position upon inflation. The interiorly positioned retaining member (14) can be in the form of a soft plastic inflatable balloon cuff combined with an exteriorly positioned retaining member in the form of a flange (16), between the two retaining members an annular space is formed. In order to inflate the inflatable element air is injected through a pilot hole using a syringe or the like into the pilot balloon (26) which has an air conduit extending from the pilot balloon to the balloon cuff i.e. the retaining member (14).

Use of this tracheotomy device is complicated by the fact that the user has to both push the device into position in the trachea opening and simultaneously pump air into the inflatable part with a syringe or the like.

A similar device is known from WO 94/15657 describing an anchorable bushing for use in lining an opening to limit the size of the opening or to serve as a guide for items insertable into the opening. The device has a particular use as a temporary or permanently installed stoma lining for use in tracheostomy patients and the device comprise an expandable member at the inner end portion which expandable member prevents the inner end portion from being withdrawn from the opening. According to this document it is also necessary to use a syringe (66) or the like to blow air into an expandable membrane (44).

WO 2007/142812 relates to a tracheostoma spacer with a tubular support framework. The support framework (9) can be expanded from an initial state to a supporting state of increased diameter and has fixing elements (14, 17) at the ends. In the supporting state S, the fixing elements 14-17 can be bent at an angle of 80-100 degrees and protrude beyond the outer circumference A of the support framework (9). The fixing elements 14-17 can have circular apertures (19-22) making it easier to handle the tracheostoma spacer (8). Apparently, the fixing elements are self-expanding which could make it difficult to control the positioning and the expansion of the fixing elements.

### Description of invention

The present application relates to a hole stabilizer, as claimed, constituting a passageway for fluid having a part formed as a tube having a first end positioned at an outer available position during use and a second end positioned at an inner non-available position during use and comprising fixing elements at two separated positions placed with the distance M between them in a use position. The hole stabilizer comprises an inner layer (13) constituting a tube and an outer layer (14) constituting the fixing elements which fixing elements are brought to expand diametrically when the outer layer (14) is reduced in length relative to the inner layer (13).

According to an embodiment the inner layer (13) and the outer layer (14) are locked unreleasable to each other at the second end.

According to an embodiment both the inner layer (13) and the outer layer (14) are shaped as cylindrical profiles and the inner layer (13) has an outer diameter (d1) which is smaller than the inner diameter of the outer layer (14) (d2).

According to an embodiment the outer surface of the inner layer (13) is in sliding contact with the inner surface of the outer layer (14) when the outer layer (14) is length-reduced relative to the inner layer (13).

According to an embodiment at least one of the fixing elements is constituted by 6-20 longitudinal trough-going openings (17) placed along the periphery in the outer layer (14). The length of each opening is normally 1-3 times the outer diameter of the outer layer (14).

According to an embodiment at least one of the fixing elements (15) is constituted by a length of material having increased elasticity compared to parts of the outer layer (14) not constituting fixing elements.The increased elasticity might be obtained by reducing the thickness of the material constituting the outer layer (14) to between one half and one tenth of the material of the outer layer not constituting a fixing element.

According to a second aspect the application also relates to a perforator, as claimed, for a hole stabilizer which perforator comprises
- a handle part (24);
- a cutting needle (4) secured unreleasably to the handle part (24);
wherein the hole stabilizer constitutes a passageway for fluid having an inner layer (13) formed as a tube and being attached releasably to the needle (4) before percutaneous insertion of the needle (4).

According to an embodiment the hole stabilizer is attached to the cutting needle (4) by frictional contact as frictional contact between the hole stabilizer and the cutting needle (4) prevents the hole stabilizer from moving relative to the cutting needle (4) before being subjected to an external force.

According to an embodiment the outer surface of the hole stabilizer is provided with an indicator (M1) visually indicating an insertion depth to the user of the combined device comprising both perforator and hole stabilizer. The hole stabilizer can also be provided with an indicator (M2) visually indicating a preferred insertion depth of the hole stabilizer before expanding a proximal fixing element.

According to an embodiment the cutting needle (4) directly or indirectly is connected to and influenced by a spring unit (6), which needle (4) before use is placed in a retracted position inside the housing and after use the needle (4) is placed percutaneously and at least outside the housing.

According to an embodiment the first part (1 a) of the housing is placed inside the second part (1 b) of the housing in such a way that the outer surface of the first part (1 a) slides along inner surfaces of the second part (1 b).An actuator (2) can be placed in extension of the at least two parts (1 a, 1 b) constituting at least a part of the housing in such a way that the actuator prolong the length of the perforator in a direction opposite the insertion direction.

According to an embodiment the spring unit (6) is unloaded during storage i.e. before use

According to the present invention it is not necessary to introduce a hole stabilizer after the cutting blade has been placed percutaneously. According to the present invention the hole stabilizer can be placed simultaneously with the penetrating part provided with a cutting blade used to penetrate the patient's skin.

### Description of the drawings

The invention is explained in greater detail below with reference to the accompanying drawings wherein a preferred embodiment of the invention is shown.
Fig. 1 shows a cut-through view of a needle of a perforator including a hole stabilizer in a state before attachment to a patient.
Fig. 2 shows a cut-through view of a hole stabilizer in a state after attachment to a patient.
Fig. 3A, 3B and 3C shows a second embodiment of a hole stabilizer in a cut-through view after attachment, a cut-through view before attachment and including a needle and an enlargement of a cut-through view of a locking device positioned at the handle.
Fig. 4 shows an embodiment of a manual perforator attached to a hole stabilizer.

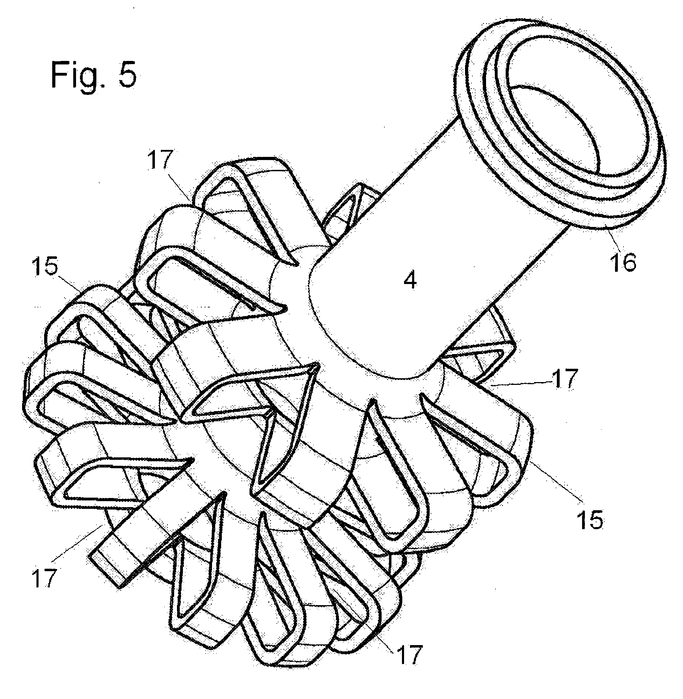

Fig. 5 shows the second embodiment of the outer layer of a hole stabilizer in a three-dimensional view in an after-attachment state.
Fig. 6 shows a perforator for controlled insertion of a hole stabilizer with insertion needle.
Fig. 7 shows the same perforator as fig. 6 with an insertion needle and a hole stabilizer mounted on the insertion needle.

After having perforated the patients skin e.g. the skin covering the trachea by establishing a tracheotomy, it is desirable to keep the opening e.g. in the form of a tracheotomy open as different layers of tissue otherwise will tend to move relative to each other which might result in that the provided opening through the tissues will disappear. Generally, the hole stabilizer can be used to provide a stationary opening and flow path from an air filled space at one side of a skin layer to the outer surface of the skin layer and the patient might be an animal or a human being.

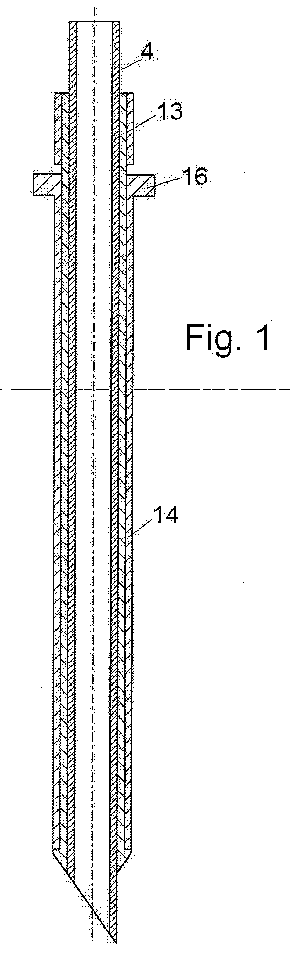

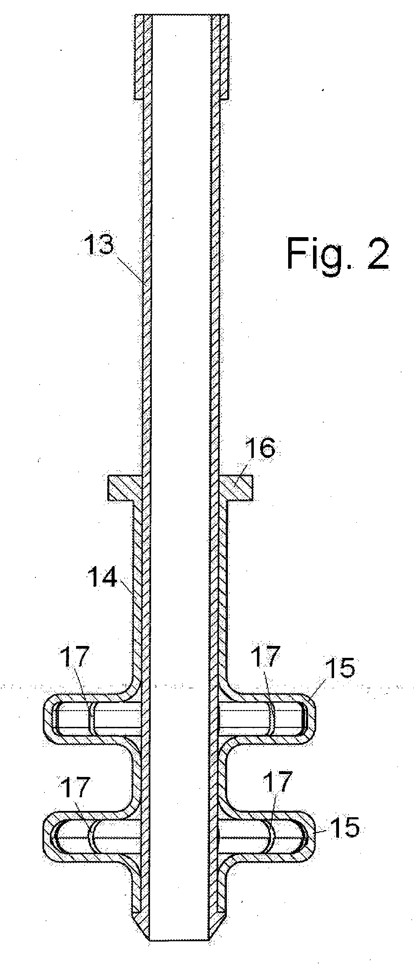

Fig. 1 and 2 illustrate an embodiment of a hole stabilizer which hole stabilizer can be inserted in the opening of the tissues simultaneously with the needle (4) of a perforator. Generally, for all embodiments of the hole stabilizer, it is possible to simultaneously cut open and insert the hole stabilizer through the cut opening as the cutting tool can be positioned in the central opening providing the flow path.

The cutting tool in the form of a hollow needle used for illustration purposes in figure 1 and 2 can be part of many different perforators both fully manual perforators and perforators having fully automated or partly automated insertion of the cutting part of the perforator. The perforator could also be solid and the cutting edge geometry could take any shape generating cutting abilities. Theoretically, the cutting tool could also surround or be placed adjecent to one side of the hole stabilizer instead of being placed at the central opening of the stabilizer.

In the shown embodiment, the hole stabilizer is only attached to the needle (4) by friction and will therefore remain in the opening of the tissues when the needle (4) of the perforator is retracted and removed, if the hole stabilizer is affected by a force larger than the frictional force between the needle and the hole stabilizer in the direction opposite of the direction in which the needle is pulled.

Fig. 1 shows the hole stabilizer in a state before use which is also the state the hole stabilizer is in during insertion through the skin layer. Fig. 2 shows the hole stabilizer in a state after insertion where the hole stabilizer works as a collar providing a close fit around the opening in the tissues while simultaneously providing an opening through which e.g. air can pass.

The shown embodiment of the hole stabilizer comprises two interconnected layers of material, an inner layer (13) and an outer layer (14). The two layers are secured to each other at the proximal end i.e. the end closest to the patient before insertion and inside the patient after insertion. The part of the outer layer (14) which is not secured to the inner layer can be displaced relative to the inner layer (13) in such a way that protruding parts (15) are formed and these protruding parts can provide a close fit around the tissue opening such that the hole stabilizing assures that e.g. air can pass uninterrupted through the tube formed by the inner layer (13) of the hole stabilizer while liquid e.g. blood leaking from the tissue is prevented or at least reduced.

The outer layer (14) of the hole stabilizer is provided with a distal collar (16) which collar (16) can be used when pushing and thereby displacing the outer layer (14) towards the proximal end after the hole stabilizer has been inserted into the patient.

Generally, the inner layer (13) is made of a relatively stiff material which remains in a given shape and the outer layer 14 is made of a more soft and elastic material which can be deformed at least in given areas. According to the shown embodiment, the material of the outer layer (14) forming the protruding parts (15) is provided with longitudinal cuts (17) which cuts allow the material to extend diametrically as the material of the outer layer is forced together i.e. reduced in length whereby the diameter of especially the central part of the protruding parts (15) is extended significantly.

Fig. 2 shows the hole stabilizer in a state of use where the stabilizer has been inserted e.g. through a tracheotomy and afterwards the movable and non-attached end of the outer layer (14) provided with the collar (16) has been forced towards the proximal end which according to the shown embodiment makes the outer layer deform into two protruding areas (15). The protruding areas (15) provide fixing elements keeping the hole stabilizer in a fixed position in the longitudinal direction of the hole stabilizer relative to the opening in the patient. The fixing elements are expanded diametrically - or in other words the fixing elements has an increased cross-section as the fixing elements need not be circular - neither in the expanded nor in the non-expanded state.

Generally, a hole stabilizer normally has two fixing elements. The externally positioned fixing element might be expanded before insertion and then it might function as an insertion stopper i.e. prevent to deep insertion of the skin penetrating needle or cutter, while the internally positioned fixing element has to be expanded after insertion of the hole stabilizer. Normally, the internally positioned fixing element will be expanded first and afterwards the externally positioned fixing element will be expanded, this embodiment assures that the two fixing elements can be firmly squeezed around the open edges of the wound as the user pushes the movable and non-attached distal end of the outer layer (14) towards the non-movable and secured proximal end of both the inner and outer layer.

As the cut-through tissue is assembled between the two protruding areas (15) of the outer layer (14), the hole stabilizer are kept in a correct position and bleeding from the cut could be prevented or at least reduced as the two protruding areas are squeezed around the wound opening.

The outer layer (14) of any embodiment of a hole stabilizer according to the invention can be provided with a handle or protruding part (16) at the distal end which handle (16) can be used when displacing the outer layer (14) relative to the inner layer (13).

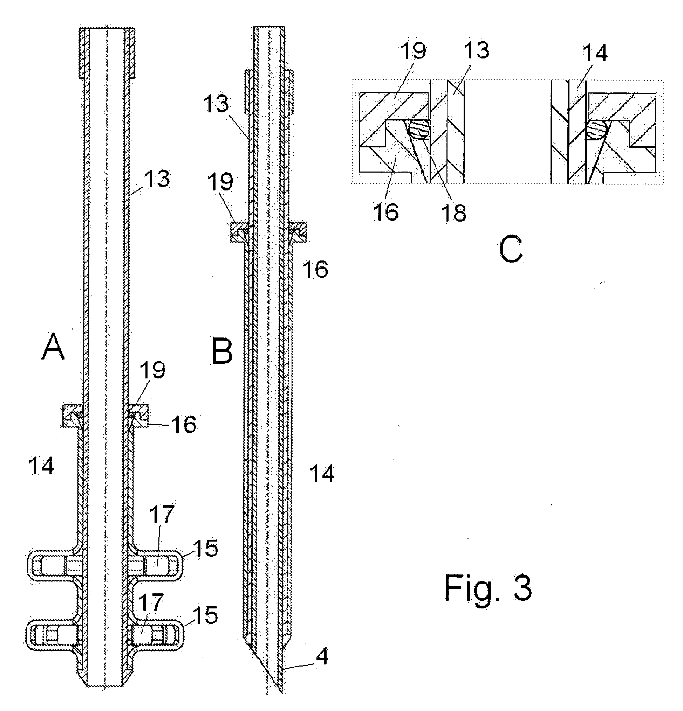

Fig. 3A, 3B and 3C shows a second embodiment of a hole stabilizer being provided with a locking device preventing that the outer layer (14) returns to the start position where it has not yet been displaced relative to the inner layer (13). Generally, it will be advantageous to provide a hole stabilizer with a locking device preventing backward movement of the outer layer (14) in the longitudinal direction when the outer layer (4) is made of an elastic material which elastic material is inclined to at least partly return to the original shape.

In order to lock the position of the outer layer (14) in a displaced position the distal end of the outer layer (14) might be provided with a locking device e.g. a friction locking device. Such a friction locking device is exemplified in fig. 3 and can consist of a ring (18) of an elastic material such as a polymer and an end cap (19) attached to the distal side of the handle (16) which is mounted at the distal end of the hole stabilizer. When forcing and sliding the end cap (19) towards the penetrating end of the perforator, the hole stabilizer establishes two protruding parts (15) acting as fixing elements, the elastic ring (18) will not prevent the movement in this direction. As the outer layer (14) of the hole stabilizer is normally made from an elastic material, the outer layer (14) will try to regain its original geometry after a deformation, i.e. it will try to return - at least to some degree - to a straight shape. Due to the conical shape of the cutout in the handle (16) the elastic ring will get trapped between the conical surface and the inner layer (13) and be pressed against the inner layer (13), thereby locking the outer layer (14) in the displaced position maintaining the protruding parts (15) of the outer layer (14).

When displacing the outer layer (14) of the hole stabilizer along the inner layer (13) in order to establish the protruding parts (15), it is in some situations desirable that the protruding part or fixing element (15) inside the patients neck - when the opening is a tracheotomy - is established first and the protruding part or fixing element (15) outside the neck is established next. Generally, predicting which fixing element extends first can e.g. be obtained by adjusting the elastic bending resistance in the material of the outer layer (14) such that the bending resistance in the element which is to extend first is lower than the bending resistance of the fixing element which is to extend second. According to the shown embodiment the bending resistance at the internally positioned fixing element is smaller than that of the externally positioned fixing element and therefore the internally positioned fixing element extends first. This decrease in the bending resistance can e.g. be established by increasing the number of longitudinal cuts (17) along the circumference of the outer layer (14) at the position where the internally positioned fixing element is based compared to the number of longitudinal cuts at the position of the externally positioned fixing element. According to the second embodiment of the hole stabilizer shown in fig. 3, the internally positioned fixing element (15) is created by cutting 12 longitudinally cuts (17) through the material of the outer layer (14) at this position, while the externally positioned fixing element (15) is created by cutting 8 longitudinal or axial cuts through the material of the outer layer at this position. The cuts are equally distributed around the circumference i.e. each band formed in each fixing element of material of the outer layer (14) has the same width.

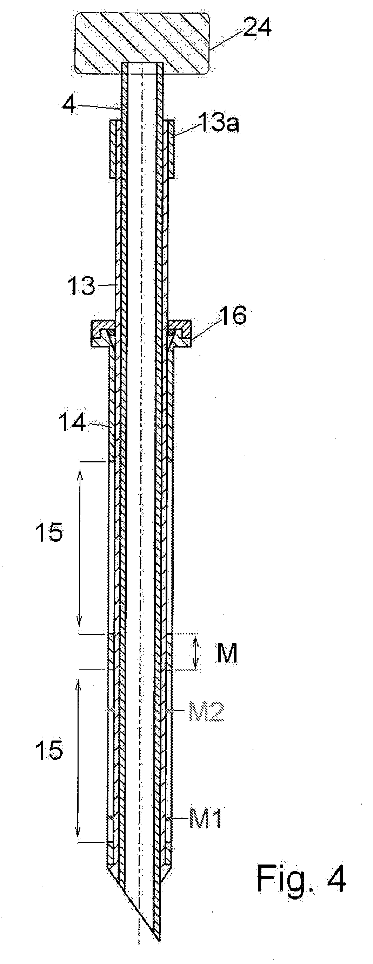

Generally, the differentiation in bending resistance can e.g. be achieved by modifying number and length of cuts, modifying the material thickness at the position of the fixing elements so that the material thickness is smaller at the position of the internally positioned fixing element compared to the thickness of the externally positioned fixing element. Further material with anisotropic properties could be used which properties result in that the material has a lower modulus of elasticity at the position of the internally positioned fixing element compared to the modulus of elasticity of the externally positioned fixing element; this variation in properties can e.g. be obtained by hardening of the material with heat or ligth.Fig. 4 shows a simple perforator which can be used to cut through the patients skin and intermediate layers into a cavity such as the trachea or another air-filled space and thereafter positioning the hole stabilizer.

Basically, the perforator comprises a cutting needle (4) provided with a cutting proximal end and a handle (24) at the opposite distal end.

Before perforation of any skin layer, the user of the perforator has to carefully consider how deep a perforator can be inserted in the air-filled space below the skin layer and the size of both hole stabilizer and perforator will depend on the specific use i.e. which skin layer is to be perforated and which air-filled space is the user gaining access to.

When opening a trachea providing a tracheotomy it should e.g.be known to the user that the anterior-posterior distance inside the larynx varies between sexes. The distance in females is typically between 13-18 mm. The distance in males is typically between 17-25 mm. The proportions of the the cutting device are limited by the anterior-posterior distance in larynx. The maximal length of the distance [End of canulla to proximal part of the distal deformation ring] is 10 mm.

The holestabilizer is i.a. designed to be used in the neck of humans. Other purposes of use are possible with a different scaling of the device. Other identified purposes are treatment of pneumothorax demanding a minor up scaling of the device to approximately 1.5:1 and a potential veterinarian use demanding an up scaling to 2:1 and probably altered proportions.

The combined perforator/hole stabilizer is kept under sterile conditions before use. Sterile conditions can e.g. be provided by a long closed tube like container made of a hard material which container has a closed end covering the cutting end of the needle (4) and an open end which open end of the container before use is covered by the handle part (24) of the perforator e.g. by providing both the container and the handle part (24) with threads so the combined perforator/hole stabilizer can be removed from the container by unscrewing the handle part (24).

In order to work with this simple perforator the hole stabilizer can be provided with two coloured areas M1 and M2 where M1 indicates how deep the cutting needle can be inserted and M2 indicates how deep the hole stabilizer has to be inserted in order to position the hole stabilizer correctly. The position of M1 depends on where on the patient the opening is to be made e.g. if the opening is to be made in the trachea the maximum insertion depth is around 10 mm and if the opening is to be made in the lung region the maximum insertion depth would be around 15 mm, and M2 indicates how deep the hole stabilizer is to be pushed before the whole inner fixing element is inside the patient. M2 depends on the dimension of the proximal fixing element of the outer layer (14).

Also, an upper handling part (13a) of the inner layer of the hole stabilizer can be indicated with a distinctive surface or colour which makes it easy for the user to recognise the position of the handling part (13a).

The following steps are to be performed when the hole stabilizer is inserted with the shown perforator:
1. The combined perforator/hole stabilizer is removed from a sterile packing.
2. The user establishes the position of where to penetrate the patiens skin and place the proximal cutting end of the perforator at this position.
3. The user pushes the perforator through the patient's skin until the indicator M1 is at level with the patient's outer skin surface.
4. The user then take hold on the hole stabilizer at the handling part (13a) and keep the hole stabilizer in this position while the perforator is pulled away from the patient by pulling in the handle (14).
5. After having removed the perforator, the hole stabilizer is pushed further down and into the patient by holding onto the handling part (13a) with one hand until the indicator M2 is at level with the patient's outer skin surface.
6. The user then grabs the handling part (16) of the outer layer of the hole stabilizer with the second hand and while the handling part (13a) of the inner layer is kept stationary, the distal end of the outer layer (14) is pushed towards the patients skin until the user feels that the hole stabilizer has a firm grib around the wound opening.

The indicators M1 and M2 can be identically positioned and also the indicators can be constituted by other elements than a distinctive colour. The indicators though may not interfere with the insertion of neither the perforator or the hole stabilizer and they should be easily recognizable by the user.

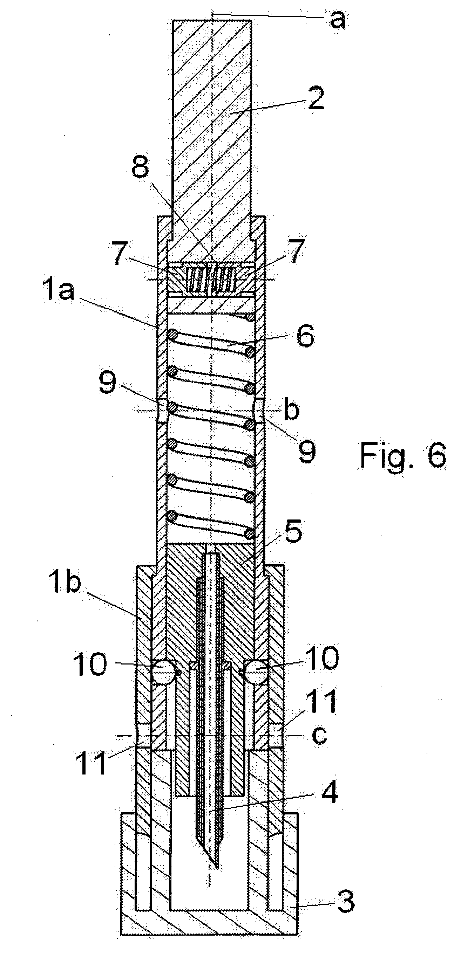

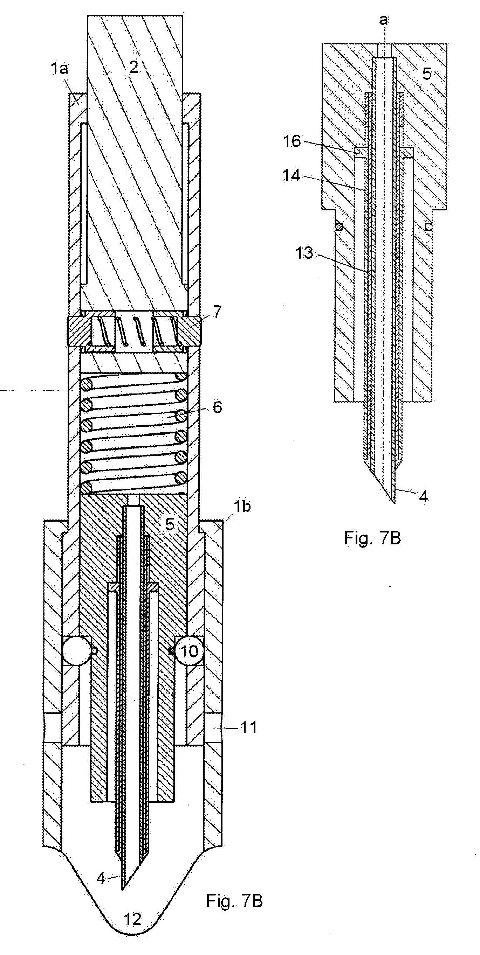

Fig. 6 and 7 illustrates two embodiments of perforators having automatic insertion of the cutting needle.

Fig. 6 shows a perforator according to the present invention in a storing state which state the perforator is in before use. The perforator comprises a housing which according to the shown embodiment is constituted by two parts (1 a) and (1 b) which two parts are secured to each other in a sliding manner ("sliding manner" means that the parts are in contact with each other but can be moved relative to each other), an actuator (2) which is placed at a first end of the perforator which one end is facing away from the patient during use, the opposite or second end rests against the patients skin during use, a needle (4) which needle (4) can penetrate the patients skin percutaneously and a closing cap (3) which protects the needle (4) before use and at the same time prevents the perforator from being actuated.

The first part (1a) of the housing i.e. the distal part relative to the patient slides relative to the second part (1 b) of the housing i.e. the proximal part relative to the patient. According to the shown embodiment both parts (1 a) and (1 b) have circular circumference and the outer surface of the first part (1a) slides along the inner surface of the second part (1b).

The friction occurring between the first part 1a and the second part (1b) is well defined and is used to control the relative movement between the two parts.

The perforator further comprises a needle hub (5) to which the needle (4) is secured and a spring (6) which spring (6) is physically placed between the actuator (2) and the needle hub (5) in such a way that the spring can push the needle hub (5) and the needle (4) towards the patient's skin. The spring (6) is illustrated as a helical spring in the figures but the "spring" could according to the present invention be any kind of unit which unit upon activation is able to provide enough force to cause the needle (4) to cut through the patient's skin and enter into a desired depth. The needle hub (5) is slide-ably attached to the first part (1a) of the housing and the needle hub can therefore move relative to this first part in the longitudinal direction i.e. the direction along the central axis a of the housing (1). The needle (4) is secured to the needle hub (5) and the needle (4) does therefore not move relative to the needle hub (5). According to the embodiment shown in fig. 6 the first part of the housing (1 a) slides inside the second part of the housing (1 b) and each part is formed as a cylinder and are able to move relative to each other along the central axis (a). The two parts might have corresponding prismatic cross-sectional areas as a prismatic cross-section will prevent the two parts from moving relative to each other in a direction around the axis a when the parts are cylindrical it is necessary e.g. to provide the contacting surfaces of the two parts with a mechanism which locks the two parts in a direction around the axis (a) e.g. the part (1a) can be provided with a longitudinal tracks in the inner surface of the outer part fitting with a protruding part on the outer surface of the inner part. The forms of the inner and outer surfaces of the first and second parts are not important; the shape of the inner and outer surfaces of the two parts should just allow the two parts to move relative to each other in a longitudinal direction in a smooth and undisturbed way while being prevented in moving around the axis (a), the part (1 a) is loaded with a small force in the longitudinal direction to overcome a well defined friction force between parts (1a) and (1b) which friction force prevent the two parts from moving relative to each other until part (1 a) is loaded with a force.

The closing cap (3) protects the needle (4) from getting into contact with the surroundings when the closing cap (3) is attached to the housing (1) because the closing cap (3) is normally made of a material which cannot be penetrated by a needle. Also, the closing cap (3) prevents the first part (1)a of the housing (1) from moving relative to the second part (1 b) of the housing. This feature prevents damaging of the needle (4) during storage because according to the shown embodiment it is only possible for the needle (4) to move relative to the first part (1 a) of the housing if the first part (1a) is moved forward relative to the second part (1b).

The perforator is provided with several locking mechanisms which allow separate parts of the perforator to move relative to each other during use without exceeding certain limits.

A first set of locking means comprise protruding parts (7) which are either attached to or a part of the actuator (2) or alternatively the protruding parts (7) are not attached to the actuator (2) but are separate parts which are moved forward together with the actuator (2) when the actuator (2) is pressed toward the patient along the axis (a). According to the embodiment shown in figs. 6-7 the locking means (7) comprise two protruding parts (7) which can be pushed outward i.e. towards and beyond the outer surface of the first part (1a) of the housing by the locking spring (8). According to other embodiments the locking means (7) might comprise one or more than two protruding parts. The locking means (7) actually locks the movement of the actuator relative to the first part (1 a) when the locking means (7) are lined with two opposite primary openings (9) which primary openings (9) are positioned in the walls of the first part (1 a) of the housing. According to the present embodiment the openings (9) are through going openings but according to other embodiments the openings (9) might comprise blind holes.

To pre-load the driving spring (6), the handle (2) is moved (pressed) along the longitudinal axis until the locking means (7) are aligned with the two openings (9). After this pre-loading the driving spring is ready for use in the penetration process.

A second set of locking means comprise balls (10) which balls (10) can move perpendicularly to the longitudinal axis a when the balls (10) passes the openings (11) in the walls of the second part 1 b of the housing. Just as the primary openings the secondary openings according to the shown embodiment are through going openings but they might be either through going openings or blind holes.

Before use the second set of locking means locks the needle hub (5) relative to the first part (1 a) of the housing. After release of the locking means which release according to the shown embodiment takes place when the balls (10) are lined with the two opposite openings (11), then the needle hub (5) can move relative to the first part (1a) of the housing. In this situation the spring (6) will drive the needle hub and the needle assembly forward to perform the penetration of tissues. The two opposite openings (11) are designed such a way that the balls (10) will pass all the way through the openings.

Fig. 7A shows the same embodiment of a perforator as shown in fig. 6 but in fig. 7A the perforator is in a pre-loaded state. Fig. 7B shows an enlargement of the needle hub (5). In the pre-loaded state the actuator (2) has been pressed toward the first part (1 a) of the housing and by this movement the first locking means has been activated as the protruding parts (7) has been pushed into the opposite openings (9) of the first part (1a) of the housing.

The lower edge of the device is shaped with two oppositely positioned protruding parts (12) which protruding parts (12) allow the user to watch the non-inserted needle end before insertion through the opening formed between the two protruding parts (12). This means that the user will be able to position the device more precisely. Alternatively, the lower edge of the second part (1 b) could be made of a transparent material or the lower edge could be provided with a single opening, both alternatives would allow the user to see the end of the needle (4) before insertion.

After having perforated the patients skin e.g. the skin covering the trachea by establishing a tracheotomy, it is a good idea to keep the hole e.g. in the form of a tracheotomy open as different layers of tissue will move relative to each other which might result in that the provided opening through the tissues will disappear.

The hole stabilizer described in this application may be scaled in size to allow for anatomic diversity. Further the invention may also be used for veterinary purposes.

| **List of references:** | |
|---|---|
| Housing | 1 |
| First part of housing | 1a |
| Second part of housing | 1b |
| Actuator | 2 |
| Closing cap | 3 |
| Needle | 4 |
| Needle hub | 5 |
| Spring | 6 |
| Protruding parts | 7 |
| Locking spring | 8 |
| Primary openings in first part | 9 |
| Balls of second locking means | 10 |
| Secondary openings | 11 |
| Protruding parts of lower edge of second part of housing | 12 |
| Inner layer / air supply tube | 13 |
| Distal handling part of inner layer | 13a |
| Outer layer / | 14 |
| Protruding parts of hole stabilizer / fixing elements | 15 |
| Collar or handle of hole stabilizer | 16 |
| Longitudinal cuts in protruding parts 15 | 17 |
| Tight ring of elastic material | 18 |
| End cap | 19 |

## Claims

1. A hole stabilizer constituting a passageway for fluid having a part formed as a tube having a first end positioned at an outer available position during use and a second end positioned at an inner non-available position during use and comprising fixing elements (15) at two separated positions placed with the distance M between them in a use position, **characterized in that** the hole stabilizer comprises an inner layer (13) constituting a tube and an outer layer (14) constituting the fixing elements (15) which fixing elements are brought to expand diametrically when the outer layer (14) is reduced in length relative to the inner layer (13).

2. A hole stabilizer according to claim 1, wherein the inner layer (13) and the outer layer (14) are locked un-releasable to each other at the second end.

3. A hole stabilizer according to claim 1 or 2, wherein both the inner layer (13) and the outer layer (14) are shaped as cylindrical profiles and the inner layer (13) has an outer diameter (d1) which is smaller than the inner diameter of the outer layer (14) (d2).

4. A hole stabilizer according to claim 1, 2 or 3, wherein the outer surface of the inner layer (13) is in sliding contact with at least a part of the inner surface of the outer layer (14) when the outer layer (14) is length-reduced relative to the inner layer (13).

5. A hole stabilizer according to any of the preceding claims, wherein at least one of the fixing elements is constituted by 6-20 longitudinal trough-going openings (17) placed along the periphery in the outer layer (14).

6. A hole stabilizer according to claim 5, wherein the length of each opening is 2-4 times the outer diameter of the outer layer (14).

7. A hole stabilizer according to claim 1, 2, 3 or 4, wherein at least one of the fixing elements (15) is constituted by a length of material having increased elasticity compared to parts of the outer layer (14) not constituting fixing elements.

8. A hole stabilizer according to claim 7, wherein the increased elasticity is obtained by reducing the thickness of the material constituting the outer layer (14) to between one half and one tenth of the material of the outer layer not constituting a fixing element.

9. A perforator comprising a hole stabilizer according to claim 1-8, which perforator comprises
- a handle part (24);
- a cutting needle (4) secured un-releasable to the handle part (24);
wherein the hole stabilizer constituting a passageway for fluid having an inner layer (13) formed as a tube is attached releasable to the needle (4) before percutaneous insertion of the needle (4).

10. A perforator, for a hole stabilizer, according to claim 9, wherein the hole stabilizer is attached to the cutting needle (4) by frictional contact as frictional contact between the hole stabilizer and the cutting needle (4) prevents the hole stabilizer from moving relative to the cutting needle (4) before being subjected to an external force.

11. A perforator, for a hole stabilizer, according to claim 9 or 10, wherein the outer surface of the hole stabilizer is provided with an indicator (M1) visually indicating an insertion depth to the user.

12. A perforator according to claim 9, 10 or 11, wherein the cutting needle (4) directly or indirectly is connected to and influenced by a spring unit (6), which needle (4) before use is placed in a retracted position inside the housing and after use the needle (4) is placed percutaneously and at least outside the housing.

13. A perforator according to claim 12, wherein a first part (1a) of the housing is placed inside a second part (1b) of the housing in such a way that the outer surface of the first part (1 a) slides along inner surfaces of the second part (1b).

14. A perforator according to claim 13, wherein an actuator (2) is placed in extension of the at least two parts (1a, 1b) constituting at least a part of the housing in such a way that the actuator prolong the length of the perforator in a direction opposite the insertion direction.

15. A perforator according to claim 12, 13 or 14, wherein the spring unit (6) is unloaded during storage i.e. before use.

## Patentansprüche

1. Lochstabilisator, welcher einen Durchgang für Fluid bildet und ein als ein Rohr ausgebildetes Teil mit einem ersten Ende, welches während des Gebrauchs in einer äußeren, verfügbaren Position angeordnet ist, und einem zweiten Ende, welches während des Gebrauchs in einer inneren, nicht verfügbaren Position angeordnet ist, aufweist, und welcher Befestigungselemente (15) in zwei gesonderten Positionen umfasst, welche in einer Gebrauchsposition um den Abstand M zwischen ihnen angeordnet sind, **dadurch gekennzeichnet, dass** der Lochstabilisator eine ein Rohr bildende Innenschicht (13) und eine die Befestigungselemente (15) bildende Außenschicht (14) aufweist, welche Befestigungselemente dazu veranlasst werden, sich diametrisch zu erweitern, wenn die Außenschicht (14) relativ zur Innenschicht (13) in der Länge reduziert wird.

2. Lochstabilisator nach Anspruch 1, wobei die Innenschicht (13) und die Außenschicht (14) am zweiten Ende miteinander unlösbar verriegelt sind.

3. Lochstabilisator nach Anspruch 1 oder 2, wobei sowohl die Innenschicht (13) als auch die Außenschicht (14) als zylindrische Profile geformt sind, und die Innenschicht (13) einen Außendurchmesser (d1) aufweist, welcher kleiner als der Innendurchmesser der Außenschicht (14) (d2) ist.

4. Lochstabilisator nach Anspruch 1, 2 oder 3, wobei sich die Außenfläche der Innenschicht (13) mit zumindest einem Teil der Innenfläche der Außenschicht (14) in Gleitkontakt befindet, wenn die Außenschicht (14) relativ zur Innenschicht (13) in der Länge reduziert wird.

5. Lochstabilisator nach einem der vorgehenden Ansprüche, wobei zumindest eines der Befestigungselemente durch 6-20 längliche, durchgehende Öffnungen (17) ausgebildet ist, welche entlang der Peripherie in der Außenschicht (14) angeordnet sind.

6. Lochstabilisator nach Anspruch 5, wobei die Länge jeder Öffnung das 2-4-fache des Außendurchmessers der Außenschicht (14) beträgt.

7. Lochstabilisator nach Anspruch 1, 2, 3 oder 4, wobei zumindest eines der Befestigungselemente (15) durch eine Länge von Material mit einer erhöhten Elastizität im Vergleich zu Teilen der Außenschicht (14), die keine Befestigungselemente bilden, ausgebildet ist.

8. Lochstabilisator nach Anspruch 7, wobei die erhöhte Elastizität dadurch erhalten ist, dass die Stärke des die Außenschicht (14) bildenden Materials auf zwischen einer Hälfte und einem Zehntel der kein Befestigungselement bildenden Außenschicht reduziert ist.

9. Perforator umfassend einen Lochstabilisator nach Anspruch 1 bis 8, welcher Perforator umfasst
- ein Griffteil (24);
- eine an dem Griffteil (24) unlösbar befestigte Schneidnadel (4);
wobei der Lochstabilisator, welcher einen Durchgang für Fluid bildet und eine als ein Rohr ausgebildete Innenschicht (13) aufweist, vor der perkutanen Einführung der Nadel (4) an der Nadel (4) lösbar befestigt ist.

10. Perforator für einen Lochstabilisator nach Anspruch 9, wobei der Lochstabilisator durch Reibkontakt an der Schneidnadel (4) befestigt ist, da ein Reibkontakt zwischen dem Lochstabilisator und der Schneidnadel (4) den Lochstabilisator daran hindert, sich relativ zur Schneidnadel (4) zu bewegen, bevor er einer äußeren Kraft ausgesetzt wird.

11. Perforator für einen Lochstabilisator nach Anspruch 9 oder 10, wobei die Außenfläche des Lochstabilisators mit einem Anzeiger (M1) ausgestattet ist, welcher dem Benutzer eine Einführungstiefe visuell anzeigt.

12. Perforator nach Anspruch 9, 10 oder 11, wobei die Schneidnadel (4) mit einer Federeinheit (6) direkt oder indirekt verbunden ist und durch diese beeinflusst ist, welche Nadel (4) vor dem Gebrauch in einer zurückgezogenen Position innerhalb des Gehäuses angeordnet ist, und die Nadel (4) nach dem Gebrauch perkutan und zumindest außerhalb des Gehäuses angeordnet ist.

13. Perforator nach Anspruch 12, wobei ein erstes Teil (1 a) des Gehäuses innerhalb eines zweiten Teils (1 b) des Gehäuses derart angeordnet ist, dass die Außenfläche des ersten Teils (1 a) entlang von Innenflächen des zweiten Teils (1 b) gleitet.

14. Perforator nach Anspruch 13, wobei ein Aktuator (2) in Verlängerung der zumindest zwei Teile (1a, 1b), welche zumindest einen Teil des Gehäuses bilden, derart angeordnet ist, dass der Aktuator die Länge des Perforators in einer der Einführungsrichtung entgegengesetzten Richtung verlängert.

15. Perforator nach Anspruch 12, 13 oder 14, wobei die Federeinheit (6) während der Aufbewahrung, d.h. vor dem Gebrauch, unbelastet ist.

## Revendications

1. Stabilisateur de trou constituant un passage pour fluide, comportant une partie formée comme un tube ayant une première extrémité positionnée à une position extérieure disponible pendant l'utilisation et une deuxième extrémité positionnée à une position intérieure non-disponible pendant l'utilisation et comprenant des éléments de fixation (15) à deux positions séparées placées avec la distance M entre elles dans une position d'utilisation, **caractérisé en ce que** le stabilisateur de trou comprend une couche intérieure (13) constituant un tube et une couche extérieure (14) constituant les éléments de fixation (15), lesdits éléments de fixation étant amenés à s'élargir diamétralement lorsque la couche extérieure (14) est réduite en longueur par rapport à la couche intérieure (13).

2. Stabilisateur de trou selon la revendication 1, dans lequel la couche intérieure (13) et la couche extérieure (14) sont verrouillées de manière non libérable l'une à l'autre au niveau de la deuxième extrémité.

3. Stabilisateur de trou selon la revendication 1 ou 2, dans lequel à la fois la couche intérieure (13) et la couche extérieure (14) ont la forme de profilés cylindriques, et la couche intérieure (13) a un diamètre extérieur (d1) qui est inférieur au diamètre intérieur de la couche extérieure (14) (d2).

4. Stabilisateur de trou selon la revendication 1, 2 ou 3, dans lequel la surface extérieure de la couche intérieure (13) est en contact de glissement avec au moins une partie de la surface intérieure de la couche extérieure (14) lorsque la couche extérieure (14) est réduite en longueur par rapport à la couche intérieure (13).

5. Stabilisateur de trou selon l'une quelconque des revendications précédentes, dans lequel au moins l'un des éléments de fixation est constitué par 6 à 20 ouvertures longitudinales traversantes (17) placées le long de la périphérie dans la couche extérieure (14).

6. Stabilisateur de trou selon la revendication 5, dans lequel la longueur de chaque ouverture est 2 à 4 fois le diamètre extérieur de la couche extérieure (14).

7. Stabilisateur de trou selon la revendication 1, 2, 3 ou 4, dans lequel au moins l'un des éléments de fixation (15) est constitué par une longueur de matériau ayant une élasticité accrue par rapport à des parties de la couche extérieure (14) ne constituant pas des éléments de fixation.

8. Stabilisateur selon la revendication 7, dans lequel l'élasticité accrue est obtenue en réduisant l'épaisseur du matériau constituant la couche extérieure (14) à une épaisseur comprise entre une moitié et un dixième du matériau de la couche extérieure ne constituant pas un élément de fixation.

9. Perforateur comprenant un stabilisateur de trou selon la revendication 1 à 8, perforateur qui comprend
- une partie de poignée (24);
- une aiguille de coupe (4) fixée de manière non-libérable à la partie de poignée (24);
dans lequel le stabilisateur de trou constituant un passage pour fluide ayant une couche intérieure (13) formée comme un tube est attaché de manière libérable à l'aiguille (4) avant l'insertion percutanée de l'aiguille (4).

10. Perforateur pour un stabilisateur de trou selon la revendication 9, dans lequel le stabilisateur est attaché à l'aiguille de coupe (4) par contact de friction, le contact de friction entre le stabilisateur de trou et l'aiguille de coupe (4) empêchant le stabilisateur de trou de se déplacer par rapport à l'aiguille de coupe (4) avant d'être soumis à une force extérieure.

11. Perforateur pour un stabilisateur de trou selon la revendication 9 ou 10, dans lequel la surface extérieure du stabilisateur de trou est pourvue d'un indicateur (M1) indiquant visuellement une profondeur d'insertion à l'utilisateur.

12. Perforateur selon la revendication 9, 10 ou 11, dans lequel l'aiguille de coupe (4) directement ou indirectement est raccordée à et influencée par une unité de ressort (6), ladite aiguille (4), avant l'utilisation, étant placée dans une position rétractée à l'intérieur du logement et l'aiguille (4), après l'utilisation, étant placée de façon percutanée et au moins à l'extérieur du logement.

13. Perforateur selon la revendication 12, dans lequel une première partie (1 a) du logement est placée à l'intérieur d'une deuxième partie (1 b) du logement si bien que la surface extérieure de la première partie (1 a) glisse le long des surfaces intérieures de la deuxième partie (1 b).

14. Perforateur selon la revendication 13, dans lequel un actionneur (2) est placé dans le prolongement des au moins deux parties (1a, 1 b) constituant au moins une partie du logement si bien que l'actionneur prolonge la longueur du perforateur dans une direction opposée à la direction d'insertion.

15. Perforateur selon la revendication 12, 13 ou 14, dans lequel l'unité de ressort (6) est déchargée pendant le stockage, c'est-à-dire avant l'utilisation.
